(19)

Europäisches
Patentamt
European
Patent Office
Office européen
des brevets

(11)  **EP 4 512 524 A1**

(12)  **EUROPEAN PATENT APPLICATION**
published in accordance with Art. 153(4) EPC

(43) Date of publication:
**26.02.2025  Bulletin 2025/09**

(21) Application number: **24799110.2**

(22) Date of filing: **18.06.2024**

(51) International Patent Classification (IPC):
*B01J 29/83* (2006.01)   *C07C 7/148* (2006.01)
*C07C 11/02* (2006.01)   *B01J 29/40* (2006.01)
*B01J 29/08* (2006.01)   *B01J 29/70* (2006.01)
*B01J 29/18* (2006.01)   *B01J 37/02* (2006.01)
*B01J 37/04* (2006.01)   *B01J 37/08* (2006.01)

(52) Cooperative Patent Classification (CPC):
**B01J 29/08; B01J 29/18; B01J 29/40; B01J 29/70;
B01J 29/83; B01J 37/02; B01J 37/04; B01J 37/08;
C07C 7/148; C07C 11/02**

(86) International application number:
**PCT/KR2024/008367**

(87) International publication number:
**WO 2025/005569 (02.01.2025 Gazette 2025/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.06.2023  KR 20230084858**

(71) Applicant: **Korea Research Institute of Chemical
Technology
Daejeon 34114 (KR)**

(72) Inventors:
• **KANG, Na Young
  Daejeon 34114 (KR)**
• **PARK, Yong Ki
  Daejeon 34114 (KR)**

• **SHIN, Ji Ho
  Daejeon 34114 (KR)**
• **KIM, Do Kyoung
  Daejeon 34114 (KR)**
• **KIM, Eun Sang
  Daejeon 34114 (KR)**
• **JUNG, Tae Young
  Daejeon 34114 (KR)**
• **LEE, Hong Jin
  Daejeon 34114 (KR)**
• **MOON, Won Kyung
  Daejeon 34114 (KR)**

(74) Representative: **Beck & Rössig
European Patent Attorneys
Denninger Str. 169
81925 München (DE)**

(54)  **CATALYST FOR PRODUCING BASIC CHEMICAL FEEDSTOCK FROM DIESEL**

(57)  The present invention relates to a catalyst for converting diesel and kerosene, which are increasingly likely to be not utilized, into basic chemical raw materials, and a method for producing the catalyst, and the catalyst is a catalyst containing a porous zeolite having pores; and phosphorus (P) supported in internal pores of the zeolite and on a surface of the zeolite, wherein the amount of Lewis acid sites in the phosphorus-supported zeolite is 20 to 100 $\mu$mol/g, and the catalyst is used for producing a basic chemical raw material through catalytic cracking of a hydrocarbon feed having a boiling point of 150°C or higher and 550°C or lower.

**EP 4 512 524 A1**

**FIG. 1**

**Description**

[Technical Field]

The present invention relates to a catalyst for producing light olefins and BTX as basic chemical raw materials by catalytic cracking of diesel and kerosene, a method for producing the same, and a use thereof.

[Background Art]

Diesel is a mixture of hydrocarbons having a boiling point of 250 to 350°C and is mainly used as fuel for passenger cars, buses, trucks, small and medium-sized ships, and large passenger aircraft. Recently, in accordance with a rapid increase in demand for electric vehicles due to global warming, a global environmental issue, the associated transportation powertrains change, and alternative renewable energies are developed, and accordingly, a demand for heavier fractions than naphtha, such as diesel and kerosene, is expected to stagnate or decrease. In addition, the European Union (EU) is pushing ahead with a plan to completely ban the sale of vehicles with gasoline and diesel engines as early as 14 years later. Accordingly, there is a need to develop a technology that may selectively produce light olefins, which are basic chemical raw materials, through a cracking reaction of a diesel fraction, the amount of which is expected to decrease in oil refineries in the future due to non-utilization.

A catalyst technology for converting a diesel fraction into a basic chemical raw material has not yet been commercialized, since a pyrolysis reaction itself, which induces lightening through a cracking reaction, is a strongly endothermic reaction, a high temperature reaction with 500 degrees or higher is required, and when a solid acid catalyst is used, the amount of energy used to activate cracking performance may be reduced, and therefore, a cracking technology using a catalyst may be selected as an excellent candidate technology.

Among similar catalytic technologies, the paper "Steam catalytic cracking of n-dodecane over Ni and Ni/Co Bimetallic catalyst supported on Hierarchical BEA zeolite" (Energy Fuels 2017, vol 31, p 5482-5490), which reports on a BEA zeolite-based catalytic cracking reaction technology using n-dodecane as a model compound of heavy oil, describes the advantages of a catalytic technology in which silica in zeolite is dissolved with strong alkali to convert a structure to a hierarchical structure that may three-dimensionally connect pores in zeolite, and a metal is supported. It is reported that, in the case of using BEA having a hierarchical structure that supports cobalt, ethylene, propylene, and butene may be produced with a total yield of up to 28.6 wt% at a reaction temperature of 400 degrees. However, in the above similar prior art, the catalyst is designed by considering only the cracking characteristics of paraffin, which accounts for about 20% of the components in the diesel fraction, and there is no technical consideration for targeting the remaining hydrocarbons corresponding to naphthene and aromatics.

In addition, in the paper "Cracking performance of gasoline and diesel fractions from catalytic pyrolysis of heavy gas oil derived from Canadian synthetic crude oil" (Energy fuels 2011, vol 25, p 3382-3388), which reports on a cracking reaction technology through a catalytic pyrolysis reaction using heavy gas oil, it is reported that ethylene and propylene may be produced with a total yield of up to about 10 wt% at a reaction temperature of 700 degrees using a formed catalyst containing ZSM-5 zeolite. Although the above prior art induces a cracking reaction at a temperature higher than the existing naphtha cracking temperature, there is no description of a specific catalyst structure to improve an olefin yield, and only the possibility of converting heavy gas oil into a basic chemical raw material is stated without any technical consideration for improving catalyst characteristics.

Accordingly, the present inventors have found that the selectivity for light olefins and BTX as basic chemical raw materials from diesel having a diverse and complex structure in a range of $C_{10}$-$C_{20}$ may be increased by controlling a specific acid site in zeolite, thereby completing the present invention.

[Disclosure]

[Technical Problem]

An object of the present invention is to provide a catalyst that may maximize productivity of light olefins and BTX as basic chemical raw materials by controlling acid sites through introduction of phosphorus (P) into a specific zeolite in producing light olefins and BTX as basic chemical raw materials by catalytic cracking of diesel with a catalyst, and a method for producing the same.

In addition, another object of the present invention is to provide a catalyst that may increase the selectivity for light olefins and BTX as basic chemical raw materials by targeting hydrocarbons corresponding to naphthene and aromatics contained in hydrocarbon feeds such as diesel and kerosene with diverse and complex structures in a range of $C_{10}$-$C_{20}$ through application of a phosphorus-supported zeolite in which Lewis acid sites, Bronsted acid sites, and total acid sites are controlled to specific ranges.

[Technical Solution]

In one general aspect, a catalyst contains a porous zeolite having pores; and phosphorus (P) supported in internal pores of the zeolite and on a surface of the zeolite, wherein the amount of Lewis acid sites in the phosphorus-supported zeolite is 20 to 100 μmol/g, and the catalyst is used for producing a basic chemical raw material through catalytic cracking of a hydrocarbon feed having a boiling point of 150°C or higher and 550°C or lower.

The phosphorus (P)-supported zeolite may have the amount of total acid sites of 200 to 600 μmol/g.

The phosphorus (P)-supported zeolite may satisfy the following Expression 1.

$$[\text{Expression 1}]$$

$$3 \le A2/A1 \le 12$$

In Expression 1, A1 and A2 are the amount (μmol/g) of Lewis acid sites and the amount (μmol/g) of Bronsted acid sites in the phosphorus-supported zeolite, respectively.

The phosphorus (P)-supported zeolite may satisfy the following Expression 2.

$$[\text{Expression 2}]$$

$$0.1 \le P/Al < 1.4$$

In Expression 2, P is a content (mol) of phosphorus supported on the zeolite, and Al is a content (mol) of aluminum in the zeolite.

The zeolite may include at least one selected from the group consisting of Y, ZSM-5, ZSM-11, ZSM-22, NU-87, TNU-9, PST-32, UZM-35, mordenite, and beta having a Si/Al mole ratio of 200 or less.

The zeolite may have an average pore size of 3 to 8 Å.

The hydrocarbon feed may be kerosene, diesel, or a mixture thereof, and the basic chemical raw material may include light olefins including ethylene and propylene and BTX.

In another general aspect, a catalyst contains the phosphorus (P)-supported porous zeolite; clay; and an inorganic oxide binder, wherein the catalyst is used for producing a basic chemical raw material through catalytic cracking of a hydrocarbon feed having a boiling point of 150°C or higher and 550°C or lower in a fluidized bed reactor.

In still another aspect, a method for producing the catalyst includes: (a) mixing a porous zeolite having pores and a phosphorus (P) compound in a solvent in contents corresponding to a P/Al mole ratio of 0.1 to 1.1; (b) drying the mixture at 100 to 150°C for 5 to 20 hours; and (c) calcining a product in the drying step at 400 to 600°C for 2 to 10 hours.

The phosphorus (P) compound may include phosphoric acid, ammonium phosphate, and/or alkyl phosphate.

In still another aspect, a method for producing a basic chemical raw material includes subjecting a hydrocarbon feed having a boiling point of 150°C or higher and 550°C or lower to catalytic cracking in the presence of the catalyst.

The catalytic cracking may be performed under reaction conditions of a reaction temperature of 600 to 700°C, a reaction pressure of 0.1 to 5 bar, and a weight ratio of the catalyst to the reactant of 2 to 20.

[Advantageous Effects]

The present invention relates to a catalyst capable of converting diesel and kerosene, which are increasingly likely to be not utilized, into basic chemical raw materials, and a method for producing the catalyst, which are novel technologies capable of selectively producing light olefins and BTX, which are basic chemical raw materials, from diesel and kerosene that may be less-utilized in the future, and are expected to be commercially applied to the oil refining and petrochemical industries.

[Description of Drawings]

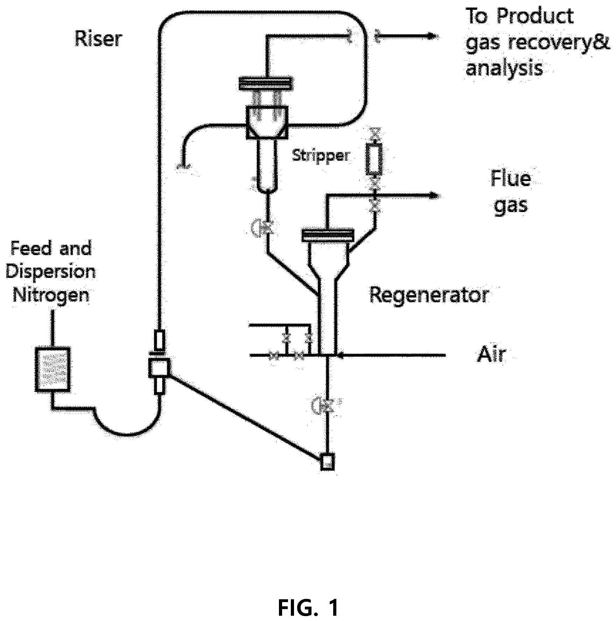

FIG. 1 is a schematic diagram of a circulating fluidized bed reactor used for producing a basic chemical raw material through a diesel catalytic cracking reaction.

[Best Mode]

Unless defined otherwise, all terms (including technical and scientific terms) used in the present specification may have the same meanings as commonly understood by those skilled in the art to which the present invention pertains.

Throughout the present specification, unless explicitly described to the contrary, "comprising" any components will be understood to imply further inclusion of other components rather than the exclusion of any other components. In addition, singular forms are intended to include plural forms, unless the context clearly indicates otherwise.

In the present specification, the "zeolite" is used with the same meaning as the "porous zeolite having pores".

An aspect of the present invention provides a catalyst used for producing light olefins and BTX, which are basic chemical raw materials, by catalytic cracking of diesel, kerosene, and the like in the presence of a catalyst according to an aspect of the present invention.

The catalyst is a catalyst containing a porous zeolite having pores; and phosphorus supported in internal pores of the zeolite and on a surface of the zeolite, wherein the amount of Lewis acid sites in the phosphorus-supported zeolite is 20 to 100 $\mu$mol/g, and the catalyst is used for producing a basic chemical raw material through catalytic cracking of a hydrocarbon feed having a boiling point of 150°C or higher and 550°C or lower.

The catalyst of the present invention is a catalyst for a catalytic cracking reaction that produces light olefins and BTX as basic chemical raw materials by catalytic cracking of diesel, kerosene, and the like, and in a solid acid catalyst such as zeolite, Bronsted and/or Lewis acid sites are formed on a solid surface, and the solid acid catalyst may be used in various processes such as catalytic cracking, isomerization, alkylation, and the like of hydrocarbon reactants in the petrochemical industry. In zeolite, silicon (Si) and aluminum (Al) are bonded to each other with oxygen as a bridge, and therefore, acid sites may be generated.

The amount of Lewis acid sites in the phosphorus-supported zeolite may be 20 to 100 $\mu$mol/g, and specifically, 20 to 50 $\mu$mol/g or 30 to 50 $\mu$mol/g. In the present invention, as the amount of Lewis acid sites in the zeolite is controlled to a specific range through phosphorus supporting, a lightening reaction of diesel, kerosene, and the like is induced, such that selectivity for light olefins and BTX may be improved. Specifically, it is required to control the Lewis acid sites, Bronsted acid sites, and the total acid sites to facilitate cracking of hydrocarbons such as iso-paraffin, naphthene, and aromatics, which are contained in high amounts in diesel, kerosene, and the like.

Meanwhile, a method for supporting phosphorus is a simple and easy method that may change the Lewis acid site in zeolite, but when synthesizing zeolite in the related art, a method of synthesizing zeolite so that an acid site is changed on its own or removing Si and Al by leaching or an acid or a base may increase process complexity, which may make it difficult to reproduce in mass production technology in actual industries.

The amount of total acid sites in the phosphorus-supported zeolite may be 200 to 600 $\mu$mol/g, and specifically, 200 to 500 $\mu$mol/g, 200 to 400 $\mu$mol/g, 200 to 300 $\mu$mol/g, or 250 to 300 $\mu$mol/g. As the amount of total acid sites in the zeolite is controlled to the above range, the production of by-products such as methane, ethane, and propane gas may be reduced, and the selectivity for light olefins and BTX may be improved.

The phosphorus-supported zeolite may satisfy the following Expression 1.

$$[Expression\ 1]$$

$$3 \leq A2/A1 \leq 12$$

In Expression 1, A1 and A2 are the amount ($\mu$mol/g) of Lewis acid sites and the amount ($\mu$mol/g) of Bronsted acid sites in the phosphorus-supported zeolite, respectively.

The phosphorus-supported zeolite may satisfy $3 \leq A2/A1 \leq 12$, $3 \leq A2/A1 \leq 10$, $3 \leq A2/A1 \leq 7.5$, or $4 \leq A2/A1 \leq 7$. In the present invention, as a ratio of the Lewis acid sites and the Bronsted acid sites in the zeolite is controlled, it is possible to target hydrocarbons corresponding to naphthene and aromatics contained with various and complex structures in a range of $C_{10}$-$C_{20}$ in hydrocarbon feeds such as diesel and kerosene, thereby increasing the selectivity for light olefins and BTX as basic chemical raw materials.

The phosphorus-supported zeolite may further satisfy the following Expression 1-1.

$$[Expression\ 1-1]$$

$$0.1 \leq A1/At \leq 0.2$$

In Expression 1-1, A1 and At are the amount ($\mu$mol/g) of Lewis acid sites and the amount ($\mu$mol/g) of total acid sites in the phosphorus-supported zeolite, respectively.

The phosphorus-supported zeolite may satisfy $0.1 \leq A1/At \leq 0.2$, $0.11 \leq A1/At \leq 0.18$, or $0.13 \leq A1/At \leq 0.17$. In the present invention, a ratio of the Lewis acid sites to the total acid sites in the zeolite is controlled, such that the effects described above may be further improved.

Meanwhile, a content of the total acid sites, a content of the Lewis acid sites, and a content of the Bronsted acid sties of the present invention may be measured by pyridine-FTIR analysis, but are not limited thereto. For example, when pyridine is adsorbed on the Bronsted acid sites in the zeolite, the pyridine accepts a proton, becomes pyridinium, and exhibits an infrared absorption band at 1,540 cm$^{-1}$, and the Lewis acid sites in the zeolite share an electron pair with the pyridine and exhibits an absorption band at 1,450 cm$^{-1}$, such that the acid contents may be distinguished according to the type of acid site.

The phosphorus-supported zeolite may satisfy the following Expression 2.

$$[Expression\ 2]$$

$$0.1 \leq P/Al < 1.4$$

In Expression 2, P is a content (mol) of phosphorus supported on the zeolite, and Al is a content (mol) of aluminum in the zeolite.

The P/Al mole ratio may be, specifically, 0.1 to 1.2, 0.1 to 1.1, 0.1 to 1.0, 0.2 to 0.9, 0.3 to 0.8, or 0.4 to 0.8. As the P/Al mole ratio of the phosphorus-supported zeolite catalyst is controlled to the above range, a catalyst suitable for the catalytic cracking of the hydrocarbon feeds such as diesel and kerosene may be designed. Light olefins and BTX may be produced with high yields in catalytic cracking of a mixture containing diesel and kerosene, and this result is analyzed to be related to the amount of Lewis acid sites in the catalyst. Meanwhile, when the content of phosphorus supported is too low, the effect of improving the hydrothermal stability by phosphorus is reduced, and the number of Lewis acid sites neutralized by phosphorus is small, and therefore, the initial acid strength of the catalyst becomes too strong, which may cause a decrease in yield of light olefins by catalytic cracking of a mixed feed. When the content of phosphorus supported is too high, the total acid sites may be neutralized excessively by phosphorus, and the pores are blocked, which may significantly reduce a specific surface area of the catalyst. Accordingly, there is a problem that the catalytic activity decreases, and the conversion of hydrocarbon feeds such as diesel and kerosene decreases.

In addition, in the present invention, in order to increase the selectivity for light olefins and BTX from diesel, kerosene, and the like, phosphorus may be supported on ZSM zeolite having the most excellent cracking performance to control the acid site, and also, through a specific range of P/Al and optimized pore design, the yield of olefins may be drastically increased while at the same time relatively decreasing the yield of by-products.

Meanwhile, in the present invention, the content of phosphorus may mean the amount (mol) of phosphorus oxide ($P_2O_5$) calculated according to the stoichiometric equivalent based on the case where all phosphorus precursors supported on zeolite are converted into phosphorus oxide ($P_2O_5$) during catalytic production.

The zeolite of the present invention may have a Si/Al mole ratio of 200 or less, and specifically, 8 to 30, 8 to 25, 8 to 20, or 8 to 15. When the amount of aluminum inside the zeolite increases excessively, the thermal and hydrothermal stability may decrease, and conversely, when the amount of silicon increases, the amount of acid sites generated by aluminum may decrease, and therefore, the catalytic activity may decrease, and also, the cost effectiveness may be poor in molecular sieve synthesis. Meanwhile, the zeolite may have an FER, MFI, MEL, BEA, CHA, or MOR structure, and zeolite having an MEL structure is more preferable because it has a high light olefin selectivity. Specifically, the zeolite of the present invention may include at least one selected from the group consisting of Y, ZSM-5, ZSM-11, ZSM-22, NU-87, TNU-9, PST-32, UZM-35, mordenite, and beta, and may be preferably ZSM-11 or ZSM-5, and most preferably ZSM-5. According to this, light olefins and BTX may be produced with high selectivity during the catalytic cracking of hydrocarbon feeds such as diesel and kerosene, and at the same time, the amount of by-products produced such as carbon monoxide and methane may be minimized.

The zeolite may have an average pore size of 3 to 8 Å, 3 to 7 Å, or 4 to 5 Å. Accordingly, a contact time between the reactant and the catalyst may be appropriately controlled, and the selectivity for light olefins and BTX may be improved. When the contact time between the reactant and the catalyst is excessively increased, the side reaction may be accelerated, which may cause a decrease in selectivity for light olefins, and conversely, when the contact time is too short, the reaction conversion may decrease, which may cause a decrease in yield of light olefins.

The hydrocarbon feed is a hydrocarbon feed having a boiling point of 150°C or higher and 550°C or lower and may be kerosene, diesel, or a mixture thereof, and the basic chemical raw material produced through catalytic cracking of the hydrocarbon feed may include light olefins including ethylene and propylene and BTX.

In general, it is analyzed that diesel and kerosene contain a large amount of naphthene, aromatics, and the like. In the present invention, through a catalyst design for targeting hydrocarbons corresponding to naphthene and aromatics, the selectivity for light olefins and BTX as basic chemical raw materials may be increased from diesel having a diverse and complex structure in a range of $C_{10}$-$C_{20}$ by controlling a specific acid site in zeolite.

Another aspect of the present invention provides a catalyst containing a phosphorus-supported porous zeolite; clay; and an inorganic oxide binder, wherein the catalyst is used for producing a basic chemical raw material through catalytic cracking of a hydrocarbon feed having a boiling point of 150°C or higher and 550°C or lower in a fluidized bed reactor.

The porous zeolite is the same as the phosphorus-supported zeolite according to an aspect of the present invention, and may be contained in an amount of 1 wt% or more and 70 wt% or less with respect to the total weight of the catalyst.

The clay may be, but is not limited to, montmorillonite, saponite, kaolin, clinoptilolite, bentonite, or a combination thereof, and may be contained in an amount of 15 wt% or more and 50 wt% or less with respect to the total weight of the catalyst. The clay is contained in the amount described above, and may contribute to improving the mechanical strength of the catalyst without contributing to a decrease in activity of the catalyst.

The inorganic oxide binder may act as a binder during catalyst formation, and may include $Al_2O_3$, $SiO_2$, $Al_2O_3$-$SiO_2$, or a combination thereof, but is not limited thereof, and may be contained in an amount of 5 wt% or more and 30 wt% or less with respect to the total weight of the catalyst. The inorganic oxide binder is contained in the amount described above, and may contribute to improving the mechanical strength of the catalyst without contributing to a decrease in activity of the catalyst.

The catalyst may further contain phosphoric acid ($P_2O_5$). The phosphoric acid may be contained in an amount of 1 wt% or more and 20 wt% or less with respect to the total weight of the catalyst. The phosphoric acid ($P_2O_5$) is further contained in the amount described above, and may be present on a surface of the inorganic oxide binder and a surface of the clay. Therefore, an acid site in the inorganic oxide binder is controlled, such that a side reaction in which CO, $CH_4$, and the like are excessively produced due to the acid site in the inorganic oxide binder in a pyrolysis reaction of a feed may be suppressed, and release of aluminum of the inorganic oxide binder may be prevented, thereby maintaining the binding performance of the inorganic oxide binder.

Still another aspect of the present invention provides a method for producing a catalyst that is used in a reaction for producing a basic chemical raw material through catalytic cracking of a hydrocarbon feed having a boiling point of 150°C or higher and 550°C or lower.

The production method includes (a) mixing a porous zeolite having pores and a phosphorus compound in a solvent in contents corresponding to a P/Al mole ratio of 0.1 to 1.4; (b) drying the mixture at 100 to 150°C for 5 to 20 hours; and (c) calcining a product in the drying step at 400 to 600°C for 2 to 10 hours.

The method for producing a catalyst may be a method for producing a catalyst that is used in a reaction for producing light olefins by catalytic cracking of the mixture containing diesel and methanol according to an aspect of the present invention.

The step (a) is a step of mixing a porous zeolite having pores and a phosphorus compound to support the phosphorus compound, and the mixing is performed in a solvent so that a P/Al mole ratio is 0.1 to 1.4.

The porous zeolite having pores and the P/Al mole ratio are as described above.

The phosphorus compound basically contains phosphorus (P) in its chemical structure and may be a compound capable of reacting with the acid site in the zeolite. For example, the phosphorus compound may include phosphoric acid, ammonium phosphate, and/or alkyl phosphate, and specifically, may include phosphoric acid ($H_3PO_4$), $H_2NH_4PO_4$, $H(NH_4)_2PO_4$, and/or an organic phosphorus compound (organic phosphite) with a size capable of penetrating into the pores of the zeolite. Meanwhile, when sodium phosphate-based compounds such as $NaH_2PO_4$, $Na_2HPO_4$, and $Na_3PO_4$ are used, $Na^+$ cations generated during phosphorus supporting may affect the acid site in the zeolite, which is not preferable because the characteristics of the solid acid catalyst may not be properly implemented.

The solvent may be any solvent used in the art, and for example, distilled water may be used.

A method for supporting a phosphorus compound inside the pores of zeolite and/or on the surface of zeolite generally includes a method of impregnating zeolite with a solution containing a phosphorus compound, a chemical vapor deposition (CVD) method, or the like. When a chemical vapor deposition method is used, the phosphorus compound is heated so that it becomes a gas, and then the gaseous phosphorus compound diffuses into the pores of the zeolite and reacts with the acid site, which may not be suitable for application to the present invention.

The step (b) is a step of drying the mixture at 100 to 150°C for 5 to 20 hours, in which the solvent may be removed and the phosphorus compound may be uniformly supported in internal pores of the zeolite and/or on a surface of the zeolite. As the drying step is performed at 100 to 150°C or 100 to 130°C for 5 to 20 hours or 7 to 15 hours, it is possible to uniformly support the phosphorus compound in the zeolite, and it is easy to control a residence time of isobutane gas in the pores of the catalyst of the phosphorus-supported zeolite of the present invention and to control the acid site in the catalyst.

The drying step (b) may further include a step of evaporating the solvent in the mixture of the mixing step (a) in an evaporator at room temperature, and for example, a rotary evaporator may be used to evaporate the solvent, but the present invention is not limited thereto.

The calcining step (c) may be performed at a temperature of 400 to 600°C, for example, 450 to 550°C, for 2 to 10 hours or 3 to 8 hours, and a calcining temperature and a calcining time are not limited to these ranges. The amount of acid sites in the catalyst may be reduced by the chemical reaction between aluminum in the zeolite and added phosphorus, and the pore blocking phenomenon may be induced, such that the pore characteristics may change.

Still another aspect of the present invention provides a method for producing a basic chemical raw material, the method including subjecting a hydrocarbon feed having a boiling point of 150°C or higher and 550°C or lower to catalytic cracking in the presence of the catalyst of the present invention.

The reaction may be performed in a fluidized bed reactor, and may be performed under reaction conditions in which a reaction temperature is 600 to 700°C, a reaction pressure is 0.1 to 5 bar, a weight ratio of catalyst/total reactants (Cat/Oil ratio) is 5 to 50, and the reactants are injected at a rate of 100 to 500 kg/h based on the amount of hydrocarbons injected (hydrocarbon rate).

As a result of conducting studies on various catalyst compositions for producing basic chemical raw materials including light olefins and BTX through catalytic cracking of hydrocarbon feeds such as diesel and kerosene, the present inventors have invented a catalyst component that has high activity/high durability and may obtain high light olefin and BTX yields, and a method for producing the catalyst.

Hereinafter, preferred examples and comparative examples of the present invention will be described. However, each of the following examples is merely a preferred example of the present invention, and the present invention is not limited to the following examples.

**Examples**

**(Examples 1 to 5 and Comparative Example 1)**

(Preparation Example 1): Preparation of Zeolite Used in Present Invention

ZSM-5, ZSM-22, NU-87, TNU-9, PST-32, UZSM-35, and NU-88 were prepared according to (US Patent, 4,556,477 (1985), Nature 353 (1991) 417420, J. Am. Chem. Soc. 127 (2007) 10870-10885, Science 373 (2021) 104-107, US Patent, 7,922,997 (2011), Journal of Catalysis 215 (2003) 151-170, and Journal of Materials Science, 51 (2016) 3735-3749), and a commercialized product available from Albemarle Corporation was used for Y. The internal pore sizes of the respective types of zeolites are as shown in Table 1.

[Table 1]

| Zeolite type | Pore size (Å) |
| --- | --- |
| Y | $7.4 \times 7.4$ |
| ZSM-5 | $5.1 \times 5.5$, $5.3 \times 5.6$ |
| ZSM-22 | $4.6 \times 5.7$ |
| NU-87 | $4.8 \times 5.7$ |
| TNU-9 | $5.5 \times 5.6$, $5.4 \times 5.5$ |
| PST-32 | $6.4 \times 7.4$, $7.3 \times 7.8$ |
| UZM-35 | $6.4 \times 6.8$, $5.2 \times 5.8$, $5.2 \times 5.2$ |
| NU-88 | - |

(Preparation Example 2): Preparation of Phosphorus-Supported ZSM-5 Zeolite

10 g of ZSM-5 (Si/Al = 11) zeolite was uniformly mixed with an aqueous solution containing 0.2 g of phosphoric acid ($H_3PO_4$, 85%) and 3.8 g of distilled water, the solvent was removed using a rotary evaporator, and phosphoric acid was supported. The sample prepared above was dried at 110°C for 12 hours, calcined at 500°C for 5 hours, and then cooled to room temperature to prepare ZSM-5 zeolite having P/Al = 0.5, which is a ratio of a content of phosphorus (P) to a content of aluminum (Al), and the ZSM-5 zeolite was used in the present experiment (Example 1).

ZSM-11 zeolites having P/Al = 0, 0.7, 1.0, 1.2, and 1.4, which is a ratio of a content of phosphorus (P) to a content of aluminum (Al), respectively, were prepared in the same manner as above by changing the zeolite and the mole ratio of phosphoric acid (Comparative Example 1 and Examples 2 to 5).

(Preparation Example 3): Preparation of Spherical Catalyst for Fluidized Bed Reaction Using Phosphorus-Supported ZSM-5 Zeolite

A molecular sieve slurry was prepared by slowly adding 32.65 kg of a ZSM-5 (Si/Al=11) molecular sieve to 64.24 kg of distilled water under stirring, 14.3 kg of 85% phosphoric acid was additionally added, and then stirring was performed at room temperature for 30 minutes. Thereafter, a solution in which 55 kg of alumina sol ($Al_2O_3$ content 10 wt%) was dispersed was additionally added and stirred for 1 hour, and then, 33.3 kg of clay was added and thoroughly mixed for 2 hours using a high-viscosity slurry mixer. The slurry was subjected to spray forming to obtain a microsphere catalyst having a particle size of 75 to 200 μm, the obtained formed catalyst was calcined at 700°C for 5 hours, and in order to confirm the hydrothermal stability, the prepared sample was charged into a reactor, distilled water was injected at a rate of 0.3 kg/h using a liquid pump and vaporized, and the distilled water was brought into contact with the sample in the form of steam. The hydrothermal treatment was performed at 760°C for 24 hours in a 100% steam atmosphere.

**Experimental Examples**

[Experimental Example 1] Production of Basic Chemical Raw Material through Diesel Catalytic Cracking Reaction By Type of Zeolite

0.1 g of the catalyst prepared in Preparation Example 1 of the present invention was charged into a fixed bed reactor, diesel was injected at a rate of 2.1 ml/h, and a reaction was performed at a reaction temperature of 600°C and a reaction pressure of 1 bar. The composition of the diesel used in Example 1 is as shown in Table 2, and the results of the catalytic cracking reaction of the diesel are summarized in Table 3.

[Table 2]

| Carbon No. | n-Paraffin | iso-Paraffin | Olefin | Naphthene | Aromatics | Total |
|---|---|---|---|---|---|---|
| $C_6$-$C_8$ | 0.4 | 0.7 | 0.2 | 1.9 | 1.3 | 4.4 |
| $C_9$-$C_{12}$ | 3.1 | 9.5 | 2.2 | 25.3 | 7.6 | 47.8 |
| $C_{13}$-$C_{19}$ | 7.4 | 14.9 | 2.9 | 15.9 | 2.9 | 41.7 |
| $C_{19}$-$C_{32}$ | 2.3 | 1.6 | 0 | 0 | 0 | 6 |
| Sum | 13.2 | 26.7 | 5.2 | 43. 1 | 11.7 | 99.9 |
| (In Table 2, a unit is wt%) | | | | | | |

[Table 3]

| Zeolite type | | Thermal | Y | ZSM-5 | ZSM-22 | NU-87 | TNU-9 | PST-32 | UZM-35 | NU-88 |
|---|---|---|---|---|---|---|---|---|---|---|
| Conversion (wt%) | | 49 | 52.3 | 69.5 | 51.1 | 55.4 | 55.4 | 48.4 | 48.3 | 58.3 |
| Selectivity (wt%) | E+P | 32.9 | 34.1 | 18.2 | 37.6 | 37.5 | 34.4 | 31.4 | 30.2 | 34 |
| | BTX | 23 | 26.7 | 72.7 | 28.2 | 29.8 | 36.8 | 19.8 | 26.2 | 36.3 |
| Yield (wt%) | E+P | 16.1 | 17.8 | 12.6 | 19.2 | 20.8 | 19.1 | 15.2 | 14.6 | 19.8 |
| | BTX | 11.3 | 14.0 | 50.5 | 14.4 | 16.5 | 20.4 | 9.6 | 12.7 | 21.2 |
| (In Table 3, E is $C_2H_4$ and P is $C_3H_6$) | | | | | | | | | | |

From the results in Table 3, in order to determine zeolite suitable for a diesel catalytic cracking reaction, several types of zeolites having an average pore size of 5 Å were selected by considering a kinetic diameter of each of the diesel components in Table 2, and Example 1 was performed. From the results in Table 3, it was confirmed that, except for UZSM-35, when a catalyst was used, diesel was converted into a basic chemical raw material by lightening more effectively than a pyrolysis reaction without a catalyst, and ZSM-5 zeolite showed the highest conversion and the highest selectivity for light olefins and BTX products.

[Experiment Example 2] Production of Basic Chemical Raw Material through Diesel Catalytic Cracking Reaction Using Phosphorus-Supported ZSM-5 Zeolite

(Evaluation Methods)

* Measurement of Amount of Lewis Acid Sites, Amount of Bronsted Acid Sites, and Amount of Total Acid Sites

In order to analyze the total acid site content, the amount of Bronsted acid sites, and the amount of Lewis acid sites of the present invention, pyridine-FTIR analysis was performed using Nicolet6700 model equipment available from Thermo Fisher Scientific Inc. 0.01 g of a measurement sample was placed in a mold and evenly distributed, and a pressure of 3 tons was applied, thereby preparing pellets. In order to measure an IR spectrum at a high temperature and in vacuum, an in-situ IR cell formed of stainless steel and $CaF_2$ window were used, the IR cell was heated to 300°C under reduced pressure and then pretreated for 1 hour, and then pyridine steam was adsorbed on the sample at 100°C for 30 minutes. Thereafter, the physically adsorbed pyridine was removed by vacuum decompression at the same temperature and analysis was performed at 150°C. When pyridine is adsorbed on the Bronsted acid sites in the zeolite, the pyridine accepts a proton, becomes pyridinium, and exhibits an infrared absorption band at 1,540 cm$^{-1}$, and the Lewis acid sites in the zeolite share an electron pair with the pyridine and exhibits an absorption band at 1,450 cm$^{-1}$. Accordingly, the acid amounts were distinguished according to the type of acid site. The results are summarized in Table 4.

\* Evaluation of Catalytic Cracking Reaction of Diesel

0.1 g of each of the catalysts prepared in Examples 1 to 5 and Comparative Example 1 was charged into a fixed bed reactor, diesel was injected at a rate of 2.1 ml/h, and a reaction was performed at a reaction temperature of 600°C and a reaction pressure of 1 bar. The composition of the diesel used in Example 1 is as shown in Table 2, and the results of the diesel catalytic cracking reaction are summarized in Table 5.

[Table 4]

|  | P/Al (mole ratio) | Amount of Bronsted acid sites (A2) | Amount of Lewis acid sites (A1) | Amount of total acid sites (At) | A2/A1 Ratio | A1/At Ratio |
|---|---|---|---|---|---|---|
| Comparative Example 1 | 0 | 454 | 90 | 560 | 5.0 | 0.16 |
| Example 1 | 0.5 | 241.6 | 48.6 | 290.3 | 5.0 | 0.17 |
| Example 2 | 0.7 | 236.8 | 34.9 | 271.7 | 6.8 | 0.13 |
| Example 3 | 1 | 219.6 | 22 | 241.6 | 10.0 | 0.09 |
| Example 4 | 1.2 | 130.6 | 16 | 146.5 | 8.1 | 0.11 |
| Example 5 | 1.4 | 68 | 10 | 78 | 6.8 | 0.13 |
| (In Table 4, a unit of the amount of acid sites is $\mu$mol/g) | | | | | | |

[Table 5]

|  | Comparative Example 1 | Example 1 | Example 2 | Example 3 | Example 4 | Example 5 |
|---|---|---|---|---|---|---|
| P/Al | 0.0 | 0.5 | 0.7 | 1 | 1.2 | 1.4 |
| wt % | | | | | | |
| $CH_4$ | 4.7 | 3.3 | 2.9 | 2.3 | 2.2 | 2.0 |
| $C_2H_6$ | 6.2 | 4.5 | 4.3 | 3.2 | 3.0 | 2.7 |
| $C_2H_4$ | 7.6 | 10.2 | 11. 6 | 10. 4 | 8.5 | 9.1 |
| $C_3H_8$ | 5.1 | 4.5 | 3.7 | 1.8 | 0.9 | 0.8 |
| $C_3H_6$ | 5.0 | 9.6 | 12.0 | 13.9 | 12.5 | 8.2 |
| $C_4H_{10}$ | 0.1 | 0.3 | 0.3 | 0.2 | 0.1 | 0.0 |
| $C_4H_8$ | 0.6 | 1.5 | 2.0 | 2.7 | 2.8 | 1.9 |
| E+P | 12.6 | 19.8 | 23.6 | 24.3 | 21.0 | 17.3 |
| BTX | 50.5 | 41.3 | 35.1 | 25.0 | 16.5 | 12.5 |
| Conversion | 69.5 | 68.1 | 67.0 | 60.6 | 49.9 | 49.0 |

Through the catalytic cracking reaction results of Comparative Example 1 and Examples 1 to 5, it was confirmed that, as the content of phosphorus increased compared to the content of aluminum in the zeolite, the amount of total acid sites and the amount of Lewis acid sites decreased and the side reactions were suppressed, and therefore, the gas components of alkanes (methane, ethane, and propane) gradually decreased, and the yield of light olefins, which are more cost-effective than BTX among the basic chemical fractions, gradually increased.

Meanwhile, through the catalytic cracking reaction results of Examples 4 and 5, it was confirmed that, when the amount of Lewis acid sites in the zeolite was lost 70% or more (corresponding to P/A= 1.2, 1.4), the conversion of diesel decreased to a level similar to that of a simple pyrolysis reaction, and thus, it was difficult to effectively induce a lightening reaction as a catalyst.

[Experimental Example 3] Production of Basic Chemical Raw Material through Diesel Catalytic Cracking Reaction Based on Fluidized Bed Reaction

Based on the results of Experimental Example 2, a circulating fluidized bed reactor, as illustrated in the schematic diagram of FIG. 1, was prepared to produce a basic chemical raw material from diesel using the spherical formed catalyst obtained in Example 1. The circulating fluidized bed reactor includes a riser (a section where a catalytic cracking reaction between diesel and a catalyst is performed), a regenerator (a section where a deactivated catalyst is regenerated after the catalytic cracking reaction between the diesel and the catalyst), and a stripper (a section where a product produced after the catalytic cracking reaction between the diesel and the catalyst and the catalyst are separated), a length of the reactor is 7 m, and a diameter of the reactor is 1/2 inch. The fluidized bed operating conditions and reaction results are summarized in Table 6.

[Table 6]

|  | CASE 1 | CASE 2 | CASE 3 |
|---|---|---|---|
| Riser Top (°C) | 635 | 654 | 673 |
| Riser BTM (°C) | 661 | 678 | 696 |
| Cat Inlet T (°C) | 690 | 710 | 730 |
| Diesel Feed Temp (°C) | 500 | 500 | 500 |
| Delta T @ Riser | 26 | 24 | 23 |
| Dilution Ratio | 0.61 | 0.62 | 0.62 |
| Cat/Oil ratio | 32.1 | 33.7 | 29.8 |
| Residence Time (sec) | 2.72 | 2.6 | 2.51 |
| System Pressure (psig) | 25 | 25 | 25 |
| HC Feed (kg/hr) | 0.6 | 0.6 | 0.6 |
| Dilution Steam Ratio | 0.29 | 0.29 | 0.29 |
| Steam feed (kg/hr) | 171.4 | 171 | 171.1 |
| Detail Composition (Yield on Product, wt%) | | | |
| Coke (Flue) | 0.67 | 0.71 | 1.06 |
| $CO, CO_2$ | 0 | 0 | 0 |
| Hydrogen $H_2$ | 0.31 | 0.36 | 0.36 |
| Methane | 4.65 | 6.41 | 6.76 |
| Ethane | 3.44 | 4.15 | 4.37 |
| Ethylene | 14.76 | 16.89 | 17.8 |
| Propane | 2.64 | 2.22 | 2.33 |
| Propylene | 18.14 | 18.72 | 19.73 |
| $C_4$ | 9.2 | 8.25 | 8.69 |
| $C_5$&$C_6$NA | 2.33 | 1.99 | 1.68 |
| Benzene | 3.7 | 4.37 | 4.67 |
| Toluene | 7.34 | 7.83 | 8.21 |
| Xylene | 5.39 | 5.32 | 4.87 |
| $C_7$&$C_8$NA | 1.48 | 0.92 | 0.3 |

(continued)

| Detail Composition (Yield on Product, wt%) | | | |
|---|---|---|---|
| $C_9^+$ | 8.19 | 7.98 | 8.55 |

When the temperature of the riser top, which is a reaction section, was changed from 635°C to 673°C under the condition in which a catalyst circulation amount was maintained at about C/O (Cat/Oil ratio) = 30, the yield of the light olefins increased from about 32.9 wt% to 37.5 wt%, and at the same time, BTX was also produced at about 17 wt% or more. As a result, it was confirmed that a basic chemical raw material was effectively produced through the diesel catalytic cracking reaction using the catalyst developed by the present invention.

Although embodiments of the present invention have been described, the present invention is not limited to the embodiments, but may be prepared in various different forms, and it will be apparent to those skilled in the art to which the present invention pertains that the embodiments may be implemented in other specific forms without departing from the spirit or essential feature of the present invention. Therefore, it is to be understood that the exemplary embodiments described hereinabove are illustrative rather than restrictive in all aspects.

**Claims**

1. A catalyst comprising:

   a porous zeolite having pores; and
   phosphorus (P) supported in internal pores of the zeolite and on a surface of the zeolite,
   wherein the amount of Lewis acid sites in the phosphorus-supported zeolite is 20 to 100 $\mu$mol/g, and
   the catalyst is used for producing a basic chemical raw material through catalytic cracking of a hydrocarbon feed having a boiling point of 150°C or higher and 550°C or lower.

2. The catalyst of claim 1, wherein the phosphorus (P)-supported zeolite has the amount of total acid sites of 200 to 600 $\mu$mol/g.

3. The catalyst of claim 1, wherein the phosphorus (P)-supported zeolite satisfies the following Expression 1:

$$[Expression\ 1]$$

$$3 \leq A2/A1 \leq 12$$

in Expression 1, A1 and A2 are the amount ($\mu$mol/g) of Lewis acid sites and the amount ($\mu$mol/g) of Bronsted acid sites in the phosphorus-supported zeolite, respectively.

4. The catalyst of claim 1, wherein the phosphorus (P)-supported zeolite satisfies the following Expression 2:

$$[Expression\ 2]$$

$$0.1 \leq P/Al < 1.4$$

in Expression 2, P is a content (mol) of phosphorus supported on the zeolite, and Al is a content (mol) of aluminum in the zeolite.

5. The catalyst of claim 1, wherein the zeolite includes at least one selected from the group consisting of Y, ZSM-5, ZSM-11, ZSM-22, NU-87, TNU-9, PST-32, UZM-35, mordenite, and beta having a Si/Al mole ratio of 200 or less.

6. The catalyst of claim 1, wherein the zeolite has an average pore size of 3 to 8 Å.

7. The catalyst of claim 1, wherein the hydrocarbon feed is kerosene, diesel, or a mixture thereof, and the basic chemical raw material includes light olefins including ethylene and propylene and BTX.

8. A catalyst comprising:

the phosphorus (P)-supported porous zeolite of claim 1; clay; and an inorganic oxide binder,
wherein the catalyst is used for producing a basic chemical raw material through catalytic cracking of a hydrocarbon feed having a boiling point of 150°C or higher and 550°C or lower in a fluidized bed reactor.

9. A method for producing the catalyst of claim 1, the method comprising:

(a) mixing a porous zeolite having pores and a phosphorus (P) compound in a solvent in contents corresponding to a P/Al mole ratio of 0.1 to 1.1;
(b) drying the mixture at 100 to 150°C for 5 to 20 hours; and
(c) calcining a product in the drying step at 400 to 600°C for 2 to 10 hours.

10. The method of claim 9, wherein the phosphorus (P) compound includes phosphoric acid, ammonium phosphate, and/or alkyl phosphate.

11. A method for producing a basic chemical raw material, the method comprising subjecting a hydrocarbon feed having a boiling point of 150°C or higher and 550°C or lower to catalytic cracking in the presence of the catalyst of claim 1.

12. The method of claim 11, wherein the catalytic cracking is performed under reaction conditions of a reaction temperature of 600 to 700°C, a reaction pressure of 0.1 to 5 bar, and a weight ratio of the catalyst to the reactant of 2 to 20.

FIG. 1

| INTERNATIONAL SEARCH REPORT | International application No. |
|---|---|
| | **PCT/KR2024/008367** |

**A. CLASSIFICATION OF SUBJECT MATTER**

**B01J 29/83**(2006.01)i; **C07C 7/148**(2006.01)i; **C07C 11/02**(2006.01)i; **B01J 29/40**(2006.01)i; **B01J 29/08**(2006.01)i; **B01J 29/70**(2006.01)i; **B01J 29/18**(2006.01)i; **B01J 37/02**(2006.01)i; **B01J 37/04**(2006.01)i; **B01J 37/08**(2006.01)i

According to International Patent Classification (IPC) or to both national classification and IPC

**B. FIELDS SEARCHED**

Minimum documentation searched (classification system followed by classification symbols)

B01J 29/83(2006.01); B01J 21/00(2006.01); B01J 27/16(2006.01); B01J 29/18(2006.01); B01J 29/40(2006.01); B01J 29/70(2006.01); B01J 38/10(2006.01); C01B 39/36(2006.01); C10G 45/02(2006.01); C10G 61/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 제올라이트(zeolite), 인(phosphorus, P), 루이스 산점(lewis acid site), 촉매(catalyst), 디젤(diesel), 케로센(kerocene), 접촉분해(catalytic cracking)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2018-0040071 A (KOREA RESEARCH INSTITUTE OF CHEMICAL TECHNOLOGY) 19 April 2018 (2018-04-19)<br>See paragraphs [0025]-[0102]; claim 1; and example 1. | 1-12 |
| Y | JP 2013-209282 A (TOSOH CORP.) 10 October 2013 (2013-10-10)<br>See paragraphs [0052] and [0053]. | 1-12 |
| Y | US 2022-0213395 A1 (SAUDI ARABIAN OIL COMPANY) 07 July 2022 (2022-07-07)<br>See paragraphs [0023]-[0034]; and claims 1-17. | 1-12 |
| A | KR 10-0979580 B1 (SK ENERGY CO., LTD. et al.) 01 September 2010 (2010-09-01)<br>See paragraphs [0023]-[0051]; and claim 1. | 1-12 |
| A | JP 2018-183773 A (MITSUBISHI CHEMICAL HOLDINGS CORP.) 22 November 2018 (2018-11-22)<br>See paragraphs [0024]-[0027]; and claims 1-8. | 1-12 |

☐ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **19 September 2024** | **19 September 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

International application No.

**PCT/KR2024/008367**

| Patent document cited in search report | | | Publication date (day/month/year) | Patent family member(s) | | | Publication date (day/month/year) |
|---|---|---|---|---|---|---|---|
| KR | 10-2018-0040071 | A | 19 April 2018 | KR | 10-1965876 | B1 | 03 April 2019 |
| JP | 2013-209282 | A | 10 October 2013 | JP | 6229274 | B2 | 15 November 2017 |
| US | 2022-0213395 | A1 | 07 July 2022 | US | 11807818 | B2 | 07 November 2023 |
| | | | | WO | 2022-150265 | A1 | 14 July 2022 |
| KR | 10-0979580 | B1 | 01 September 2010 | BR | PI0905929 | A2 | 23 June 2015 |
| | | | | BR | PI0905929 | B1 | 26 December 2017 |
| | | | | CA | 2715171 | A1 | 13 August 2009 |
| | | | | CA | 2715171 | C | 26 April 2016 |
| | | | | CN | 101939095 | A | 05 January 2011 |
| | | | | CN | 101939095 | B | 29 May 2013 |
| | | | | EP | 2248582 | A2 | 10 November 2010 |
| | | | | EP | 2248582 | B1 | 13 November 2019 |
| | | | | ES | 2773074 | T3 | 09 July 2020 |
| | | | | JP | 2011-523584 | A | 18 August 2011 |
| | | | | JP | 5415457 | B2 | 12 February 2014 |
| | | | | MY | 160528 | A | 15 March 2017 |
| | | | | RU | 2010132886 | A | 20 March 2012 |
| | | | | RU | 2494809 | C2 | 10 October 2013 |
| | | | | SA | 109300097 | B1 | 15 December 2013 |
| | | | | TW | 200940695 | A | 01 October 2009 |
| | | | | TW | I447219 | B | 01 August 2014 |
| | | | | US | 2011-0039688 | A1 | 17 February 2011 |
| | | | | US | 2014-0213431 | A1 | 31 July 2014 |
| | | | | US | 8673802 | B2 | 18 March 2014 |
| | | | | US | 9056308 | B2 | 16 June 2015 |
| | | | | WO | 2009-099309 | A2 | 13 August 2009 |
| | | | | WO | 2009-099309 | A3 | 12 November 2009 |
| JP | 2018-183773 | A | 22 November 2018 | JP | 7087636 | B2 | 21 June 2022 |

**EP 4 512 524 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4556477 A

- US 7922997 B

**Non-patent literature cited in the description**

- Steam catalytic cracking of n-dodecane over Ni and Ni/Co Bimetallic catalyst supported on Hierarchical BEA zeolite. *Energy Fuels*, 2017, vol. 31, 5482-5490
- Cracking performance of gasoline and diesel fractions from catalytic pyrolysis of heavy gas oil derived from Canadian synthetic crude oil. *Energy fuels*, 2011, vol. 25, 3382-3388

- *Nature*, 1991, vol. 353, 417420
- *J. Am. Chem. Soc.*, 2007, vol. 127, 10870-10885
- *Science*, 2021, vol. 373, 104-107
- *Journal of Catalysis*, 2003, vol. 215, 151-170
- *Journal of Materials Science*, 2016, vol. 51, 3735-3749